# EUROPEAN PATENT APPLICATION

(11) **EP 2 801 332 A1**
(43) Date of publication of application: **12.11.2014**
(21) Application number: 13778956.6
(22) Date of filing: 17.04.2013
(51) Int. Cl.: A61B 18/00, A61B 18/12

(54) **SURGICAL DEVICE**

(30) Priority: 20.04.2012 US 201261636285 P
(71) Applicant: OLYMPUS MEDICAL SYSTEMS CORP., Tokyo 151-0072 (JP)
(72) Inventor: SANAI, Hideo, Shibuya-ku, Tokyo 151-0072 (JP); HATTA, Shinji, Shibuya-ku, Tokyo 151-0072 (JP)
(74) Representative: Schicker, Silvia
(86) International application number: PCT/JP2013/061382
(87) International publication number: WO 2013/157571

(57) **Abstract**

A surgical 1 apparatus includes: a treatment section 31 for treating a living tissue; an ultrasound output section 43 and a high-frequency output section 44 that generate energy for providing ultrasound vibration or high-frequency current to the treatment section 31; a liquid feeding tube 25 and a tube 21b for feeding a liquid; an opening portion 21a, provided in the treatment section 31, for feeding the liquid from the liquid feeding tube 25 and the tube 21b in order to feed the liquid to between the living tissue and the treatment section 31; a pump 47 for supplying the liquid to the liquid feeding tube 25 and the tube 21b; and a CPU 41. The CPU 41 controls the pump 47 so that in connection with stoppage of an output of the energy from the ultrasound output section 43 and the high-frequency output section 44, the liquid is supplied from the pump 47 for a predetermined period of time or in a predetermined amount after the stoppage of the output of the energy.

## Description

### Technical Field

The present invention relates to a surgical apparatus, and specifically relates to a surgical apparatus that can output at least either ultrasound vibration or high-frequency current.

### Background Art

Surgical treatment instruments are used for treatments such as coagulation or dissection of a living tissue in surgical operations. Among the surgical treatment instruments, there are those of a type that pinches a living tissue to perform treatment (what is called "scissors shape type"). Also, as surgical treatment instruments, for example, ultrasound treatment instruments that can output ultrasound vibration, and high-frequency treatment instruments that can output high-frequency current, and furthermore, in recent years, energy treatment instruments that can simultaneously output ultrasound vibration and high-frequency current have been known.

In a scissors shape-type ultrasound treatment instrument, one of members performs ultrasound vibration, and the other jaw member is opened/closed relative to the one member for pinching. Also, a scissors shape-type high-frequency treatment instrument provides a bipolar output of high-frequency current using two members.

Such a treatment instrument is, for example, used for a treatment for sealing and cutting a blood vessel. A surgeon closes a scissors-shaped pinching portion to pinch a blood vessel, and turns on a predetermined switch such as a foot switch to heat the pinched blood vessel part and make moisture vaporize therefrom, whereby the surgeon can coagulate or dissect the living tissue and seal or dissect the blood vessel.

The surgeon can check a state of the treatment by opening the scissors-shaped pinching portion and viewing the sealed state of the blood vessel, and can check the cutting of the blood vessel as a result of the pinching portion being moved away from the blood vessel.

For example, the specifications of US Patent Application Laid-Open Publications Nos. US2010/085196A1, US2003/0040672A1 and US2010/0324458A1 each disclose a surgical apparatus that performs treatment using a high-frequency output.

However, when the coagulation or dissection of the living tissue is performed by such an energy treatment instrument, there often arises a case where a pinched part of the living tissue is stuck to a treatment section.

Normally, surfaces of two pinching members of the treatment section are subjected to water repellant coating and the sticking of the living tissue to the treatment section is prevented. However, there is a case where the water repellant coating on the treatment section peels off after repeating the treatment and stains by burning, etc. adhere to the surfaces of the treatment section, so that the living tissue is stuck to the treatment section.

If the living tissue is stuck to the treatment section, the surgeon cannot confirm completion of the treatment. Specifically, if the living tissue is stuck to the treatment section, the surgeon cannot view a treatment state of the living tissue by opening the scissors-shaped pinching portion. Further, if the living tissue is stuck to the treatment section, a cut blood vessel does not separate from the pinching portion and therefore the surgeon cannot confirm the cutting of the blood vessel.

Further, since the living tissue is stuck to the treatment section and the blood vessel or the like does not separate from the treatment instrument although the dissection treatment is finished, there is a case where the surgeon considers that the dissection treatment is not finished and continues the output, to cause a problem that wear of pads of the treatment section progresses.

The problem that the living tissue is stuck to the treatment section and the surgeon cannot confirm the completion of the treatment when an energy output for the treatment is terminated is not taken into consideration in the above three US Patent Application Publications.

The present invention has been made in view of the above problems and an object of the present invention is to provide a surgical apparatus which prevents sticking of a living tissue to a treatment section when an energy output by ultrasound vibration or high-frequency current is terminated.

### Disclosure of Invention

### Means for Solving the Problem

A surgical apparatus according to an aspect of the present invention includes: a treatment section for treating a living tissue; an energy generation section that generates energy for providing ultrasound vibration or high-frequency current to the treatment section; a liquid feeding conduit for feeding a liquid; a liquid feeding port, provided in the treatment section, for feeding the liquid from the liquid feeding conduit in order to feed the liquid to between the living tissue and the treatment section; a pump for supplying the liquid to the liquid feeding conduit; a control section that controls the pump so that in connection with stoppage of an output of the energy from the energy generation section, the liquid is supplied from the pump for a predetermined period of time or in a predetermined amount after the stoppage of the output of the energy.

### Brief Description of the Drawings

Fig. 1 is a diagram for describing a configuration of a surgical apparatus according to a first embodiment of the present invention;
Fig. 2 is a block diagram illustrating configurations of a power supply unit 12 and a liquid feeding unit 13 according to the first embodiment of the present invention;
Fig. 3 is a diagram indicating a relationship between impedance z detected by an impedance detection section 45 and liquid feeding time period Td, which is stored in a storage section 42, according to the first embodiment of the present invention;
Fig. 4 is a flowchart illustrating an example of processing performed by a CPU 41 that controls a US output section 43, an HF output section 44 and a pump drive section 46, according to the first embodiment of the present invention;
Fig. 5 is a timing chart of an output signal EOUT for an ultrasound vibration output, a high-frequency current output or a simultaneous output of ultrasound vibration and high-frequency current, and a pump drive signal POUT for a pump 47, according to the first embodiment of the present invention;
Fig. 6 is a diagram illustrating a configuration of a surgical apparatus 1A according to a second embodiment of the present invention;
Fig. 7 is a flowchart illustrating an example of processing performed by a CPU 41 that controls a US output section 43, an HF output section 44 and a pump drive section 46, according to the second embodiment of the present invention; and
Fig. 8 is a timing chart of an output signal EOUT for an ultrasound vibration output, a high-frequency current output or a simultaneous output of ultrasound vibration and high-frequency current, a pump drive signal POUT for a pump 47, and discharge of saline from an opening portion 21 a, according to the second embodiment of the present invention.

### Best Mode for Carrying Out the Invention

The present invention will be described below by means of embodiments.

### (First Embodiment)

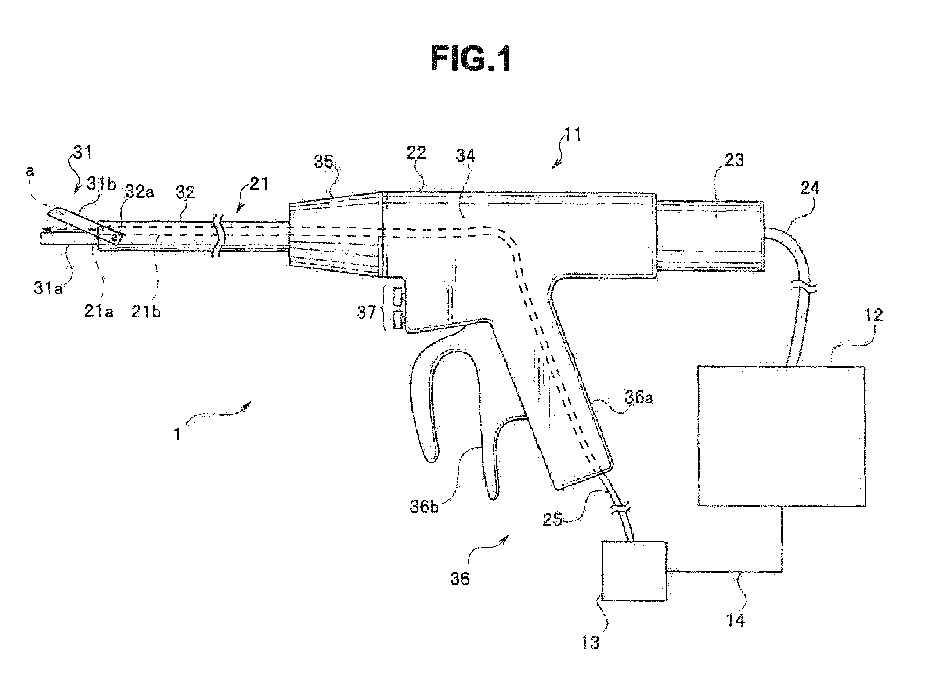

Fig. 1 is a diagram for describing a configuration of a surgical apparatus according to a first embodiment of the present invention. As illustrated in Fig. 1, a surgical apparatus 1 includes a treatment instrument 11, a power supply unit 12 and a liquid feeding unit 13.

The treatment instrument 11 is a scissors shape-type surgical treatment instrument that can output at least either ultrasound vibration energy or high-frequency current energy. The treatment instrument 11 includes a treatment unit 21, a handle unit 22, a transducer unit 23, a signal cable 24 and a liquid feeding tube 25.

The treatment unit 21 includes a treatment section 31 for treating a living tissue, and an elongated sheath portion 32. The treatment section 31 includes a probe 31a, and a movable member 31b, which is a jaw member. The sheath portion 32 is a cylindrical member, and a shaft member or the like for opening/closing the probe 31a and the movable member 31b relative to each other is inserted inside the sheath portion 32. The movable member 31b can pivot with a pin 32a as a pivot axis, the pin 32a being provided at a distal end of the sheath portion 32, according to a motion of the shaft member or the like by an operation of the handle unit 22. Accordingly, a distal end portion of the probe 31a and the movable member 31b form a pinching portion that pinches a living tissue.

Furthermore, at a distal end portion of the treatment unit 21, an opening portion 21 a for feeding saline is provided, and the opening portion 21a is connected to a tube 21b inserted inside the sheath portion 32. A proximal end portion of the tube 21b inserted also inside the handle unit 22 is connected to the liquid feeding tube 25, whereby the tube 21b and the liquid feeding tube 25 are in communication with each other. As described later, the treatment instrument 11 is configured so that saline, which is a liquid fed from the liquid feeding unit 13, can pass through the liquid feeding tube 25 and the tube 21b, and be ejected from the opening portion 21 a. Accordingly, the liquid feeding tube 25 and the tube 21b form a liquid feeding conduit for feeding saline.

Also, as indicated by dotted arrow a in Fig. 1, the opening portion 21a and a tube 12b are disposed so that saline is fed and dripped toward a pinching part between the probe 31 a and the movable member 31 b in the treatment section 31. Accordingly, the opening portion 21a is a liquid feeding port, provided in the treatment section 31, for feeding saline from the tube 21 b, which is a liquid feeding conduit, to between a living tissue and the treatment section 31.

The handle unit 22 includes a rotating knob 35 on a distal end side of a cylindrical body portion 34. A surgeon can change a position of the movable member 31b around the axis relative to the probe 31a in the treatment section 31 by rotating the rotating knob 35 around an axis of the body portion 34.

The transducer unit 23 is attached to a proximal end portion of the body portion 34. The transducer unit 23 is connected to the probe 31a. The transducer unit 23 includes an ultrasound transducer (not illustrated) inside, which enables the probe 31a to perform ultrasound vibration.

The body portion 34 includes a handle portion 36, and the handle portion 36 includes a fixed handle 36a and a movable handle 36b. The handle portion 36 is an operating handle for pinching a living tissue. When a surgeon operates the movable handle 36b so as to come close to the fixed handle 36a, that is, close the handle portion 36, the movable member 31b in the treatment section 31 pivots around the pin 32a, enabling a living tissue to be pinched between the probe 31a and the movable member 31b.

Furthermore, in the body portion 34, a plurality of switches 37 for output operation are provided. Accordingly, the surgeon turns on relevant ones of the switches 37 while grasping the handle portion 36 with a living tissue pinched between the distal end portion of the probe 31 a and the movable member 31b in the treatment section 31, whereby treatment using an ultrasound vibration output, a high-frequency current output or a simultaneous output of ultrasound vibration and high-frequency current can be performed.

The power supply unit 12 is a control apparatus, and as described later, includes a control section, and performs control of ultrasound vibration output and high-frequency current output, and liquid feeding, according to operations of the switches 37 by the surgeon.

The liquid feeding unit 13 is connected to the power supply unit 12 via a signal cable 14. Also, the liquid feeding unit 13 is connected to the treatment instrument 11 via the liquid feeding tube 25 for liquid feeding, enabling saline to be fed from the liquid feeding unit 13 to the treatment instrument 11. Although an example in which saline is fed is described here, another liquid that has no effects on human bodies can be fed.

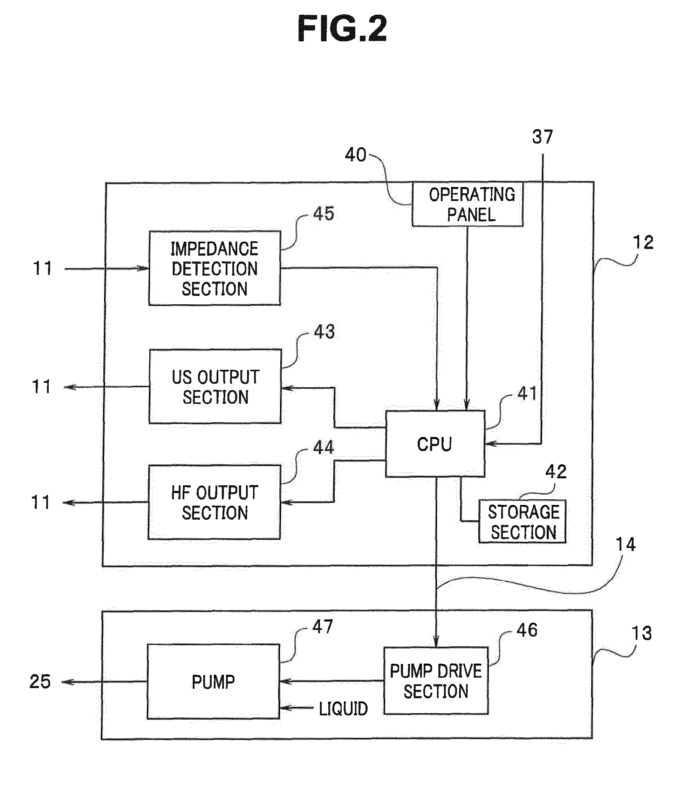

Fig. 2 is a block diagram illustrating configurations of the power supply unit 12 and the liquid feeding unit 13. The power supply unit 12 is a control apparatus that controls energy output of treatment instrument 11. The power supply unit 12 includes an operating panel 40, which serves as an operating/setting section, a central processing unit (hereinafter referred to as "CPU") 41, which serves as a control section, a storage section 42, an ultrasound output section (hereinafter referred to as "US output section") 43 that outputs a drive signal for driving the transducer unit 23 for ultrasound vibration output, a high-frequency output section (hereinafter referred to as "HF output section") 44 that outputs a high-frequency current signal for high-frequency current output, and an impedance detection section 45.

As described above, the CPU 41 controls an ultrasound vibration output, a high-frequency current output or a simultaneous output of ultrasound vibration and high-frequency current. The control is performed by the CPU 41 executing a control program stored in the storage section 42.

The storage section 42 includes, e.g., a ROM that stores the control program, a RAM that serves as a working memory area at the time of execution of the program, and a nonvolatile rewritable memory that stores information on liquid feeding time periods, which will be described later.

The US output section 43 outputs a drive signal for making the probe 31a perform ultrasound vibration, to the treatment instrument 11 via the signal cable 24 based on an ultrasound output control signal from the CPU 41.

The HF output section 44 outputs a high-frequency current signal for supplying a bipolar high-frequency output to the treatment section 31, to the treatment instrument 11 via the signal cable 24 based on a high-frequency output control signal from the CPU 41.

Accordingly, the US output section 43 and the HF output section 44 are energy generation sections that generate energy for providing ultrasound vibration and high-frequency current to the treatment section 31, respectively.

The impedance detection section 45 is a circuit for detecting an impedance of a living tissue pinched between the probe 31a and the movable member 31b in the treatment section 31. In other words, the impedance detection section 45 detects an impedance between two pinching members that pinch a living tissue in the treatment section 31. The impedance detection section 45 supplies a detection signal according to the impedance between the probe 31 a and the movable member 31 b, to the CPU 41.

Operating signals from the switches 37 are also inputted to the CPU 41. Note that here, as described later, an instruction for an energy output is provided by operation of a relevant one of the switches 37 by the surgeon; however, such energy output instruction may be provided via, e.g., a foot switch.

The liquid feeding unit 13 includes a pump drive section 46 and a pump 47. The pump drive section 46 is a drive circuit that outputs a drive signal for driving the pump 47, based on a pump drive signal from the CPU 41 via the signal cable 14. The pump 47 is connected to a non-illustrated tank, and is driven based on a drive signal from the pump drive section 46, and supplies saline retained in the tank to the tube 25. Performance of discharge by the pump 47 is, for example, 20 ml/min.

The CPU 41 controls the US output section 43 and the HF output section 44 and also controls the pump drive section 46, according to operating signals from the switches 37.

In the storage section 42, later-described data on liquid feeding time periods for saline to be fed from the pump 47, which are set in advance, is stored.

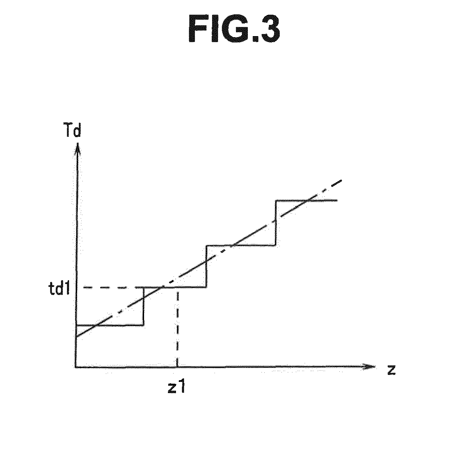

Fig. 3 is a diagram indicating a relationship between impedance z detected by the impedance detection section 45 and liquid feeding time period Td, which is stored in the storage section 42. A liquid feeding time period Td relative to an impedance z is set in advance so that as the impedance z is larger, the liquid feeding time period Td gradually increases, and stored in the storage section 42. The liquid feeding time period Td according to the impedance z is stored in the form of, for example, table data in the storage section 42.

Note that the liquid feeding time period Td may be set for each of three output types, i.e., ultrasound vibration output, high-frequency current output and simultaneous output of ultrasound vibration and high-frequency current.

Still furthermore, the liquid feeding time period Td may be set for each of types of treatment instruments 11, each of objects to be treated, or each of combinations of treatment instruments 11 and objects to be treated. In such cases, the CPU 41 reads information on an identifier (that is, ID) indicating a type of a treatment instrument 11, which is stored in, e.g., a memory provided in the treatment instrument 11, to determine the type of the treatment instrument 11. An object to be treated is selected from objects, such as the parenchyma of a liver and a blood vessel, displayed on the operating panel 40 before a surgeon uses the treatment instrument 11.

The liquid feeding time period Td according to the impedance z can be set/changed by, e.g., a surgeon via the operating panel 40 in the power supply unit 12.

A set value of the liquid feeding time period Td varies according to, e.g., the specifications of the treatment instrument 11 and/or performance of the pump 47. For example, an energy output may be large or small depending on the type and/or the output settings of the treatment instrument 11. In the case of a large energy output, the liquid feeding time period Td is long, and in the case of a small energy output, the liquid feeding time period Td is short. Accordingly, in the storage section 42, the liquid feeding time period Td according to, e.g., the specifications of each treatment instrument 11 is set.

In other words, as the liquid feeding time period Td, a period of time necessary for a pinched part of a living tissue that has been dried as a result of energy-used treatment to contain moisture sufficient for preventing the pinched part from sticking to the probe 31a or the movable member 31b in the treatment section 31 is set.

Note that in Fig. 3, the liquid feeding time period Td is set so as to increase in a stepwise manner relative to the value of the impedance z; however, as indicated by the alternate long and short dash line, the liquid feeding time period Td may be set so as to increase linearly.

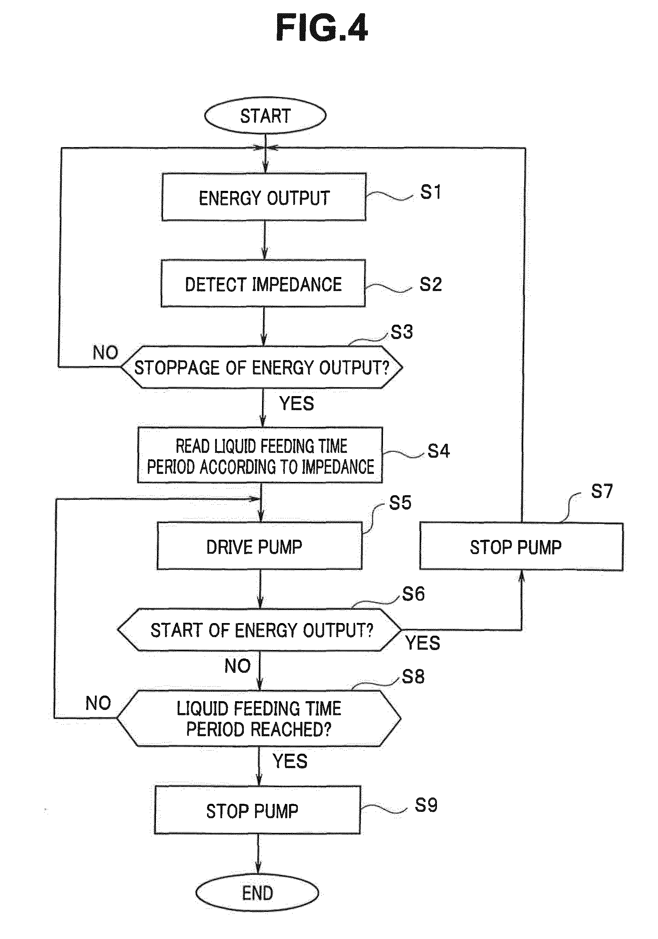

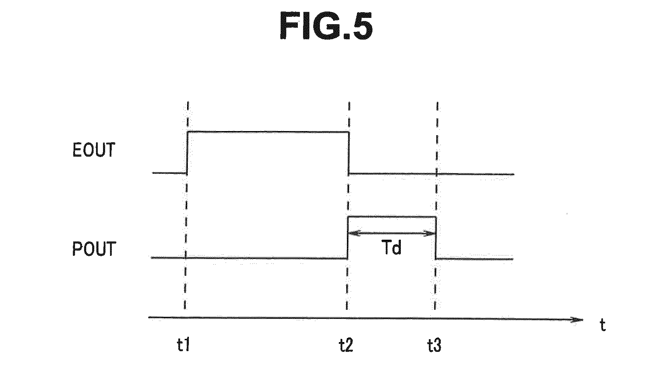

Fig. 4 is a flowchart illustrating an example of processing performed by the CPU 41 that controls the US output section 43, the HF output section 44 and the pump drive section 46. Fig. 5 is a timing chart of an output signal EOUT for an ultrasound vibration output, a high-frequency current output or a simultaneous output of ultrasound vibration and high-frequency current and a pump drive signal POUT for the pump 47.

The processing in the Fig. 4 is processing performed by the CPU 41 when a relevant one of the switches 37 is depressed and thereby turned on to provide an instruction for an energy output that is any of an ultrasound vibration output, a high-frequency current output and a simultaneous output of ultrasound vibration and high-frequency current.

Upon receipt of an instruction for an energy output provided by depression of a relevant one of the switches 37, the CPU 41 provides an energy output designated by the instruction (S1). If the energy output is an ultrasound vibration output, the US output section 43 is driven so as to provide a predetermined or designated output. If the energy output is a high-frequency current output, the HF output section 44 is driven so as to provide a predetermined or designated output. If the energy output is a simultaneous output of ultrasound vibration and high-frequency current, each of the US output section 43 and the HF output section 44 is driven so as to provide a predetermined or designated output. In Fig. 5, an output signal EOUT becomes high at a time t1, whereby energy output is started.

The CPU 41 detects an impedance z between the probe 31a and the movable member 31b based on a detection signal from the impedance detection section 45 (S2).

Then, the CPU 41 determines whether or not the switch 37 is turned off, that is, the energy output is stopped (S3), and if the energy output is not stopped (S3: NO), the processing returns to S1. If the energy output is stopped (S3: YES), the CPU 41 reads a liquid feeding time period Td stored in the storage section 42 based on the detected impedance z (S4). For example, in Fig. 3, if the impedance z is z1 (for example, 600 Ω), a value of a liquid feeding time period td1 (for example, two seconds) is read.

The CPU 41 outputs a pump drive signal POUT to the pump drive section 46 to drive the pump 47, whereby liquid feeding is performed (S5). As a result, saline is fed from the opening portion 21 a at the distal end portion of the sheath portion 32 of the treatment instrument 11, and the saline is dripped to a part of a living tissue pinched by the probe 31a and the movable member 31 b. As a result, the pinched part of the living tissue that has been dried as a result of an energy treatment receives the drip of the saline and thereby starts wetting. In Fig. 5, at a time t2, the energy output is stopped and liquid feeding is started.

The CPU 41 determines whether or not an instruction for a start of an energy output is provided by a relevant one of the switches 37 being operated when the liquid feeding is being performed (S6). If an energy output is started (S6: YES), the CPU 41 discontinues the output of the pump drive signal POUT to the pump drive section 46 to stop the pump 47, whereby the liquid feeding is stopped (S7), and the processing returns to S1. In other words, upon receipt of an instruction for generating energy to, e.g., the US output section 43, which is an energy generation section, after stoppage of an energy output, the CPU 41 stops the pump 47 to prioritize the instruction provided via a surgeon's operation.

If no energy output is started (S6: NO), whether or not a period of time passed from the start of the liquid feeding has reached the read liquid feeding time period Td is determined (S8). When an instruction for stoppage of an energy output is provided, the CPU 41 starts time measurement and determines whether or not a period of time from the start of the liquid feeding has reached the read liquid feeding time period Td, based on the value of the measurement. If the period of time passed from the start of the liquid feeding has not yet reached the liquid feeding time period Td (S8: NO), the processing returns to S5 and the liquid feeing is continued.

If the period of time passed from the start of the liquid feeding has reached the liquid feeding time period Td (S8: YES), the CPU 41 discontinues the output of the pump drive signal POUT to the pump drive section 46 to stop the liquid feeding (S9), and thereby terminate the processing. In Fig. 5, at a time t3, the liquid feeding is stopped.

In other words, the CPU 41, which is a control section, controls the pump 47 so that in connection with stoppage of an output of energy from, e.g., the US output section 43, which is an energy generation section, saline is supplied from the pump 47 for a predetermined period of time after the stoppage of the energy output. More specifically, the CPU 41 performs control so that driving of the pump 47 is started in response to stoppage of an output of energy so as to supply saline from the pump 47 and the pump 47 stops after supply of saline from the pump 47 for the predetermined period of time.

As a result, liquid feeding is performed during a set liquid feeding time period Td, and thus, a pinched part of a living tissue that has been dried as a result of vaporization of moisture due to an energy-used treatment is made to contain moisture sufficient for preventing the pinched part from sticking to the probe 31a or the movable member 31b in the treatment section 31.

If saline is dripped to a part of a living tissue that is being treated during a treatment, a problem arises in that the part that is being treated is cooled, resulting in an increase in treatment time period. However, with the above-described surgical apparatus 1 according to the present embodiment, saline is dripped after a treatment, preventing an increase in treatment time period.

Furthermore, when saline is dripped to a part of a living tissue that is being treated during a treatment, in the case of ultrasound vibration, a temperature of the saline becomes high, which may cause cavitation. If the treatment section 31 of the treatment instrument 11 comes into contact with a part of the living tissue around the part that is being treated when cavitation has occurred, the normal part of the living tissue may be damaged by the cavitation. However, with the above-described surgical apparatus 1 according to the present embodiment, saline is dripped after a treatment, preventing occurrence of such problem of cavitation.

As described above, with the above-described surgical apparatus 1 according to the present embodiment, control of liquid feeding is performed so that a living tissue that has been dried as a result of an energy treatment is made to contain moisture by saline immediately after the treatment, enabling prevention of the living tissue sticking to the treatment section 31 at the time of an end of an energy output for the treatment.

### (Second Embodiment)

Although in the above-described first embodiment, in connection with stoppage of an energy output, the CPU 41 drives the pump 47 to start liquid feeding, in a second embodiment, liquid feeding is restricted by mechanically pressing the liquid feeding tube 25 or the liquid feeding tube 21 b according to a pinching operation of the treatment section 31 provided by the handle portion 36, and liquid feeding is started when the pinching operation is cancelled.

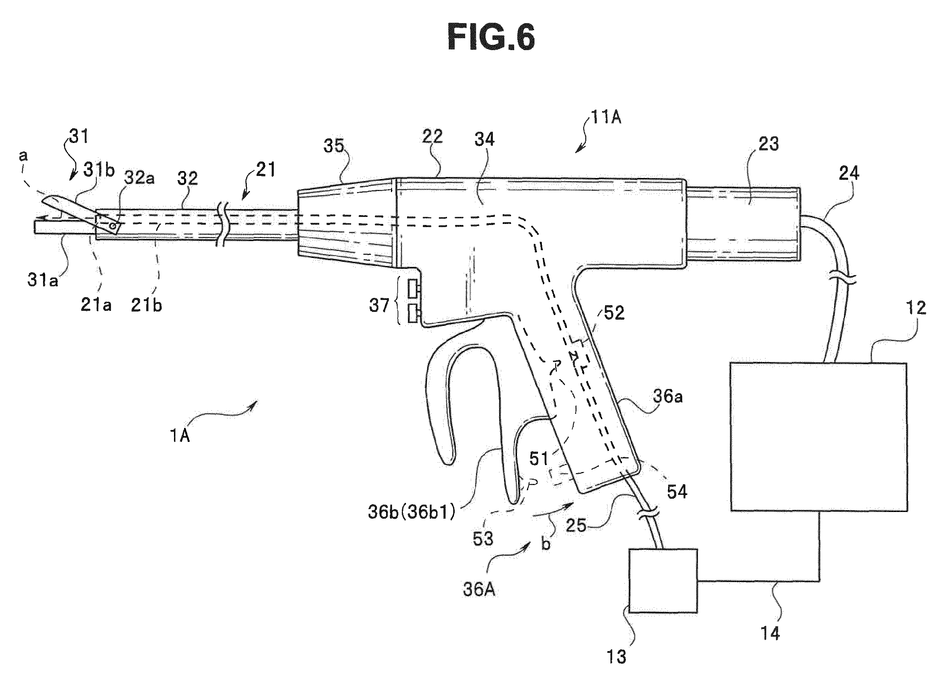

Fig. 6 is a diagram for describing a configuration of a surgical apparatus 1A according to the present embodiment. In Fig. 6, components that are the same as those in Fig. 1 are provided with reference numerals that are the same as those in Fig. 1, and a description thereof will be omitted.

In the surgical apparatus 1A, a movable handle 36b1 in a treatment instrument 11A includes a projection portion 51 that abuts against and thereby deforms a liquid feeding tube 21b when a handle portion 36A is closed. Also, inside a fixed handle 36a, a receiving member 52 that receives the liquid feeding tube 21b deformed by the projection portion 51 is provided in a fixed manner.

In other words, if the handle portion 36A is closed and the movable handle 36b1 is moved in the direction indicated by solid arrow b in Fig. 6, the projection portion 51 mechanically presses the liquid feeding tube 21b against the receiving member 52 inside the fixed handle 36a to deform the liquid feeding tube 21b so that the flow of saline flowing inside the liquid feeding tube 21b, which is fed from the pump 47, is stopped. If a surgeon opens the handle portion 36A, the liquid feeding tube 21b is released from the force from the projection portion 51, and thus, saline flows inside the liquid feeding tube 21 b.

Accordingly, the projection portion 51 and the receiving member 52 in the handle portion 36A form a liquid feeding restricting mechanism arranged at a position midway in the liquid feeding tube 21b, the liquid feeding restricting mechanism restricting feeding of a liquid flowing in the liquid feeding tube 21b in response to an operation of the movable handle 36b1, which is an operating handle for pinching a living tissue via a treatment section 31.

Configurations of a power supply unit 12 and a liquid feeding unit 13 are similar to those illustrated in Fig. 2. The content of processing performed by a CPU 41 is different from that in the first embodiment.

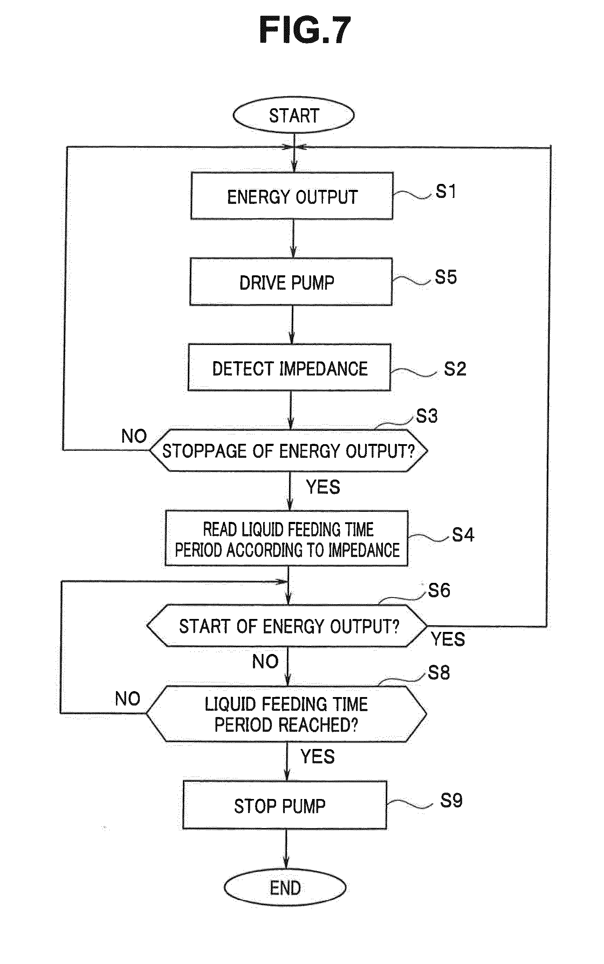

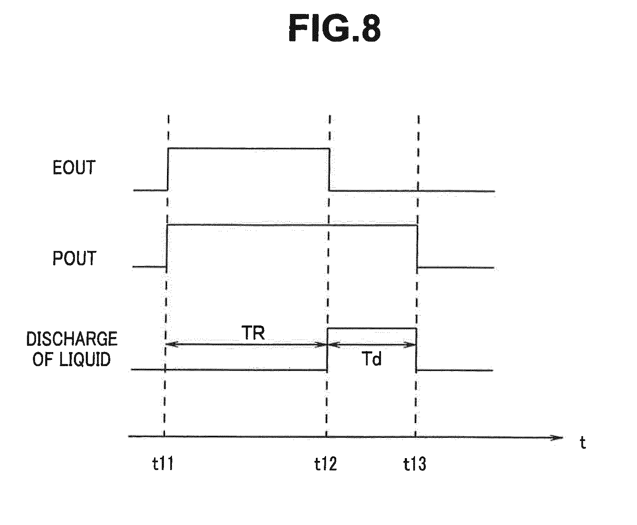

Fig. 7 is a flowchart illustrating an example of processing performed by the CPU 41 that controls the US output section 43, the HF output section 44 and the pump drive section 46. In Fig. 7, processing steps that are the same as those in Fig. 4 are provided with reference numerals that are the same as those in Fig. 4, and only a simplified description thereof will be provided. Fig. 7 is different from Fig. 4 in terms of, e.g., the order of processing steps. Fig. 8 is a timing chart of an output signal EOUT for an ultrasound vibration output, a high-frequency current output or a simultaneous output of ultrasound vibration and high-frequency current, a pump drive signal POUT for the pump 47, and discharge of saline from an opening portion 21a.

In Fig. 7, upon receipt of an instruction for an energy output provided by depression of a relevant one of switches 37, the CPU 41 provides an energy output designated by the instruction (S1), and subsequently, outputs a pump drive signal POUT to the pump drive section 46 to drive the pump 47 to perform liquid feeding (S5).

When the instruction for an energy output is provided, the handle portion 36A is closed, and thus, the projection portion 51 deforms the liquid feeding tube 21b, and therefore, even if the pump 47 is driven, the flow of saline fed from the pump 47 is stopped. In Fig. 8, an output of an output signal EOUT is started at a time t11, but feeding of saline is not performed.

Subsequent to S5, the CPU 41 detects an impedance between a probe 31a and a movable member 31 b based on a detection signal from an impedance detection section 45 (S2), and subsequently, determines whether or not the energy output is stopped (S3).

If no instruction for stopping the energy output is provided (S3: NO), the processing returns to S 1. If an instruction for stopping the energy output is provided as a result of the depression of the switch 37 being discontinued (S3: YES), the CPU 41 reads a liquid feeding time period Td according to the detected impedance z, which is stored in a storage section 42 (S4).

When the surgeon opens the handle portion 36A along with the stoppage of the energy output, the projection portion 51 no longer presses the liquid feeding tube 21b, and thus, liquid feeding is started. As a result, saline is fed from the opening portion 21 a at a distal end portion of a sheath portion 32 in the treatment instrument 11, and the saline is dripped to a part of a living tissue that has been pinched by the probe 31a and the movable member 31 b. In Fig. 8, during a time period TR from the time t11 to a time t12, the energy output is provided, and at the time t12, the energy output is stopped and liquid feeding is started.

The CPU 41 determines whether or not an instruction for a start of an energy output is provided by the switch 37 being operated when liquid feeding is performed (S6), and if an energy output is started (S6 :YES), the processing returns to S1. This is because an instruction according to a surgeon's operation is prioritized as in the first embodiment.

If no energy output is started (S6: NO), whether or not a period of time from the stoppage of the energy output has reached the read liquid feeding time period Td is determined (S8), and if the period of time passed from the start of the liquid feeding has not reached the liquid feeding time period Td yet (S8: NO), the processing returns to S6, and the liquid feeding is continued.

If the period of time passed from the start of the liquid feeding has reached the liquid feeding time period Td (S8: YES), the CPU 41 discontinues the output of the pump drive signal POUT to the pump drive section 46 to stop the liquid feeding (S9) and thereby terminates the processing. In Fig. 8, at a time t13, the liquid feeding is stopped.

Accordingly, with the present embodiment, also, liquid feeding is performed for a set liquid feeding time period Td, and thus, a pinched part of a living tissue that has been dried as a result of an energy-used treatment is made to contain moisture sufficient for preventing the pinched part from sticking to the probe 31 a or the movable member 31 b in the treatment section 31.

As described above, with the above-described surgical apparatus 1 A according to the present embodiment, control of liquid feeding is performed so that a living tissue that has been dried as a result of an energy-used treatment is made to contain moisture by saline immediately after the treatment, enabling prevention of the living tissue sticking to the treatment section 31 at the time of an end of an energy output for the treatment.

Note that although in the above-described example, an operation of the switch 37 for an energy output and an opening/closing operation of the handle portion 36A are independent from each other, a switch may be provided in the handle portion 36A so that when the switch is closed by an operation of the movable handle 36b1 in the handle portion 36A, the switch is turned on to generate an instruction signal for an energy output. For example, in Fig. 6, a projection portion 53, which is separate from the projection portion 51, is provided in the movable handle 36b1 indicated in parentheses, and a switch 54 that is pressed by the projection portion 53 when the handle portion 36A is closed is provided in the fixed handle 36a.

Such configuration enables an operation of a switch for an energy output and an opening/closing operation of the handle portion 36A to be linked to each other.

As described above, with the surgical apparatus according to each of the above-described embodiments, control of liquid feeding is performed so that a living tissue that has been dried as a result of an energy treatment is made to contain moisture by saline immediately after the treatment, enabling prevention of the living tissue sticking to the treatment section 31 at the time of an end of an energy output for the treatment.

The surgical apparatus according to each of the above-described embodiments is effective especially for energy treatment for the parenchyma of a liver. In the case of the parenchyma of a liver, which is surrounded by a membrane, when the living tissue sticks to the treatment instrument because of, e.g., the living tissue burning to the treatment instrument, if the treatment instrument is unstuck from the living tissue, heavy bleeding often occurs as a result of capillary vessels being torn apart. With the surgical apparatus according to each of the above-described embodiments, a liquid is dripped at the time of an end of an energy output for a treatment to prevent a living tissue from sticking to the treatment section 31, preventing heavy bleeding resulting from capillary vessels being torn apart.

Note that the surgical apparatus according to each of the embodiments is effectively applicable not only to the parenchyma of a liver, but also to other organs such as blood vessels.

### (Test Results)

The above two embodiments have been described on the premise that liquid feeding is started immediately after an instruction provided by a surgeon to stop an energy output. However, in a test conducted by the applicant, it has been confirmed that no sticking prevention effect is provided if liquid feeding is started after the passage of one second from an instruction for stopping an energy output. Accordingly, it is preferable to start liquid feeding in a period of time of around several milliseconds to 0.5 seconds. This is because a start of liquid feeding within such period of time enables a living tissue to contain moisture so as to prevent the living tissue from sticking to the treatment section 31.

Also, it is preferable that a liquid feeding time period be no less than 0.5 seconds and no more than 5 seconds if a discharge rate of the pump 47 is 20 ml/min.

Furthermore, the applicant conducted tests for comparison between a case where dripping is performed after an energy output described above and a case where no dripping is performed after an energy output described above. Conditions for the tests are those for a case of an ultrasound output where ultrasound is output to a carotid artery of a pig for three seconds with a bipolar output of 40 W, a frequency of 47 kHz, vibration of 80 µm and a grasping force of around 25 N. Sticking prevention coating of the treatment section in the treatment instrument came off as a result of use at a number of times in the past.

Under these conditions, five tests were conducted for each of the case where dripping of saline is performed after an energy output and the case where dripping of saline is not performed after an energy output. When a pump with feeding performance of 20 ml/min was driven for one second after stoppage of an energy output, the blood vessel did not stick to the treatment section in each of the five tests. On the other hand, when dripping of saline was not performed after stoppage of an energy output, the blood vessel stuck to the treatment section in each of the five tests.

### (Modification 1)

Although in each of the two embodiments described above, liquid feeding is performed for a predetermined period of time after an end of an energy output, it is possible that liquid feeding is performed in a predetermined amount instead of the liquid feeding being performed for a predetermined period of time. In other words, a CPU 41 may control a pump 47 so as to feed a predetermined amount of liquid necessary for a living tissue that has been dried as a result of an energy treatment to contain moisture sufficient for preventing the living tissue from sticking to a treatment instrument.

In such case, also, information on the predetermined amount, that is, a liquid feeding amount, is stored in a storage section 42, and as in the two embodiments described above, the predetermined amount is set so as to vary according to a detected impedance. Furthermore, values of the liquid feeding amounts stored in the storage section 42 can be set/changed by a surgeon.

### (Modification 2)

Although in the two embodiments and modification 1 described above, a liquid feeding time period or a liquid feeding amount is a time period or an amount set in advance according to an impedance, a surgeon may set a liquid feeding time period or a liquid feeding amount via an operating panel 40 to perform liquid feeding for the set liquid feeding time period or in the set liquid feeding amount irrespective of the impedance.

Accordingly, a CPU 41 controls a pump 47 so as to perform liquid feeding for the set liquid feeding time period or in the set liquid feeding amount after an end of an energy output.

The present invention is not limited to the above-described embodiments, and various modifications, alterations and the like are possible without departing from the spirit of the present invention.

The present application is filed claiming the priority of U.S. Provisional Application No. 61/636,285 filed in U.S. on April 20, 2012, and the above described disclosure is incorporated by reference in the present description, claims and drawings.

## Claims

1. A surgical apparatus comprising:
a treatment section for treating a living tissue;
an energy generation section that generates energy for providing ultrasound vibration or high-frequency current to the treatment section;
a liquid feeding conduit for feeding a liquid;
a liquid feeding port, provided in the treatment section, for feeding the liquid from the liquid feeding conduit in order to feed the liquid to between the living tissue and the treatment section;
a pump for supplying the liquid to the liquid feeding conduit;
a control section that controls the pump so that in connection with stoppage of an output of the energy from the energy generation section, the liquid is supplied from the pump for a predetermined period of time or in a predetermined amount after the stoppage of the output of the energy.

2. The surgical apparatus according to claim 1, wherein the predetermined period of time or the predetermined amount is stored in a storage section and is capable of being set and changed.

3. The surgical apparatus according to claim 1, comprising
an impedance detection section that detects an impedance between two pinching members that pinch the living tissue in the treatment section,
wherein the predetermined period of time or the predetermined amount varies depending on the impedance detected by the impedance detection section.

4. The surgical apparatus according to claim 1, wherein the predetermined period of time or the predetermined amount varies depending on an object to be treated.

5. The surgical apparatus according to claim 1, wherein upon receipt of an instruction for generating the energy to the energy generation section after the stoppage of the output of the energy, the control section stops the pump.

6. The surgical apparatus according to claim 1, wherein the control section performs control so that driving of the pump is started so as to perform supply of the liquid from the pump, in response to the stoppage of the output of the energy and the pump is stopped after supply of the liquid from the pump for the predetermined period of time or in the predetermined amount.

7. The surgical apparatus according to claim 1, further comprising a liquid feeding restricting mechanism arranged at a position midway in the liquid feeding conduit, the liquid feeding restricting mechanism restricting feeding of the liquid flowing in the liquid feeding conduit in response to an operation of an operating handle for pinching the living tissue via the treatment section.

8. The surgical apparatus according to claim 2, wherein the predetermined period of time is no less than 0.5 seconds and no more than 5 seconds.

9. The surgical apparatus according to claim 1, wherein the energy generated by the energy generation section is energy for ultrasound vibration, energy for high-frequency current or energy for both ultrasound vibration and high-frequency current.
